# EUROPEAN PATENT APPLICATION

(11) **EP 2 096 095 A1**
(43) Date of publication of application: **02.09.2009**
(21) Application number: 08152103.1
(22) Date of filing: 28.02.2008
(51) Int. Cl.: C07C 7/00, C07C 17/02, C07C 17/156, C07C 19/045

(54) **Process for the manufacture of at least one ethylene derivative compound**

(71) Applicant: SOLVAY (Société Anonyme), 1050 Bruxelles (BE)
(72) Inventor: The designation of the inventor has not yet been filed
(74) Representative: Jacques, Philippe

(57) **Abstract**

Process for the manufacture of at least one ethylene derivative compound starting from a low value residual gas, preferably a ROG, according to which :
a) the low value residual gas is subjected to a series of treatment steps in a low value residual gas recovery unit in order to remove the contaminants present therein and to obtain a mixture of products containing ethylene and other constituents;
b) the said mixture of products is separated into a fraction enriched with compounds which are lighter than ethylene, containing part of the ethylene (fraction A), into a fraction enriched with ethylene (fraction B) and into a heavy fraction (fraction C);
c) fraction A and fraction B are conveyed to the manufacture of at least one ethylene derivative compound.

## Description

The present invention relates to a process for the manufacture of at least one ethylene derivative compound, in particular to a process for the manufacture of 1,2-dichloroethane (DCE), a process for the manufacture of vinyl chloride (VC) and a process for the manufacture of polyvinyl chloride (PVC).

To date, ethylene which is more than 99.8 % pure is normally used for the manufacture of ethylene derivative compounds, in particular for the manufacture of DCE. This ethylene of very high purity is obtained via the cracking of various petroleum products, followed by numerous complex and expensive separation operations in order to isolate the ethylene from the other products of cracking and to obtain a product of very high purity.

Given the high costs linked to the production of ethylene of such high purity, various processes for the manufacture of ethylene derivative compounds, in particular DCE, using ethylene having a purity of less than 99.8 % have been developed. These processes have the advantage of reducing the costs by simplifying the course of separating the products resulting from the cracking and by thus abandoning complex separations which are of no benefit for the manufacture of ethylene derivative compounds, in particular DCE.

For example, patent application WO 00/26164 describes a process for the manufacture of DCE by simplified cracking of ethane coupled with chlorination of ethylene. To this effect, an ethylene chlorination step takes place in the presence of the impurities obtained during the cracking of the ethane.

Patent application WO 03/48088 describes, for its part, a process for the manufacture of DCE by dehydrogenation of ethane, giving rise to the formation of a fraction comprising ethane, ethylene and impurities including hydrogen, which fraction is then subjected to chlorination and/or oxychlorination.

The processes described have nevertheless the disadvantage that the ethylene obtained cannot be used for an ethylene chlorination/oxychlorination process given that the ethylene contains impurities whose presence during the oxychlorination reaction could cause problems of exploitation, namely a poisoning of the catalyst with heavy products and an uneconomical conversion of the hydrogen present.

Further, patent applications WO 2006/067188, WO 2006/067190, WO 2006/067191, WO 2006/067192, WO 2006/067193 and WO 2007/147870 describe a process for the manufacture of DCE starting from a hydrocarbon source, in particular naphtha, gas oil, natural gas liquid, ethane, propane, butane, isobutane or mixtures thereof, which is first subjected to a simplified cracking. Patent applications WO2008/000705, WO2008/000702 and WO2008/000693 describe, for their part, a process for the manufacture of DCE starting from a stream of ethane which is first subjected to a catalytic oxydehydrogenation. In those two groups of patent applications, two different factions containing ethylene are afterwards separated from the gas mixture issued from the simplified cracking or from the catalytic oxydehydrogenation, before being conveyed independently to a chlorination reactor and to an oxychlorination reactor.

The processes described in the above-mentioned patent applications, the aim of which is to produce and use ethylene having a purity of less than 99.8 %, are based on sources which are essentially saturated hydrocarbons which require the first steps of cracking or of dehydrogenation, in particular catalytic oxydehydrogenation, in order to produce a gaseous mixture containing insaturated hydrocarbons like ethylene. Such steps of cracking and dehydrogenation present however the disadvantage of needing an important investment which causes an increase in the production costs. Moreover these processes involve the use of expensive hydrocarbon sources.

The aim of the present invention, for its part, is to provide a process for the manufacture of at least one ethylene derivative compound, in particular for the manufacture of DCE, using ethylene with a purity of less than 99.8 % in order to reduce the high costs linked to the production of ethylene of high purity by using low value residual gas containing ethylene avoiding the need to chemically produce the ethylene from an expensive hydrocarbon source and abandoning complex separations for isolating the ethylene from the other products contained in the aforementioned residual gas which are of no benefit for the manufacture of ethylene derivative compounds, in particular DCE, and which further has the advantages of avoiding the abovementioned problems for the known processes using such quality of ethylene.

To this effect, the invention relates to a process for the manufacture of at least one ethylene derivative compound starting from a low value residual gas according to which :
a) the low value residual gas is subjected to a series of treatment steps in a low value residual gas recovery unit in order to remove the contaminants present therein and to obtain a mixture of products containing ethylene and other constituents;
b) the said mixture of products is separated into a fraction enriched with compounds which are lighter than ethylene, containing part of the ethylene (fraction A), into a fraction enriched with ethylene (fraction B) and into a heavy fraction (fraction C);
c) fraction A and fraction B are conveyed to the manufacture of at least one ethylene derivative compound.

The expression "at least one ethylene derivative compound" is understood to mean, for the purpose of the present invention, that one or more than one ethylene derivative compounds may be manufactured by the process according to the present invention.

The process according to the invention is preferably a process for the manufacture of one ethylene derivative compound.

The expression "one ethylene derivative compound" is understood to mean, for the purpose of the present invention, any compound which may be manufactured starting with ethylene.

As examples of such compounds, may be cited among others, ethylene oxide (afterwards converted to glycols), linear alpha-olefines, linear primary alcohols, homopolymers and copolymers of ethylene, ethylbenzene (afterwards converted to styrene, itself converted to polymers of styrene), vinyl acetate, acetaldehyde, ethyl alcohol, propionaldehyde and DCE (used afterwards for the manufacture of VC, itself used for the manufacture of PVC).

The process according to the invention is more preferably a process for the manufacture of one ethylene derivative compound which is DCE.

The process according to the invention is therefore more preferably a process for the manufacture of DCE starting from a low value residual gas according to which :
a) the low value residual gas is subjected to a series of treatment steps in a low value residual gas recovery unit in order to remove the contaminants present therein and to obtain a mixture of products containing ethylene and other constituents;
b) the said mixture of products is separated into a fraction enriched with compounds which are lighter than ethylene, containing part of the ethylene (fraction A), into a fraction enriched with ethylene (fraction B) and into a heavy fraction (fraction C);
c) fraction A is conveyed to a chlorination reactor and fraction B to an oxychlorination reactor, in which reactors most of the ethylene present in the fractions A and B is converted to DCE; and
d) the DCE obtained is separated from the streams of products derived from the chlorination and oxychlorination reactors.

The process according to the invention is a process starting from a low value residual gas.

The expression "a low value residual gas" (LVRG), used hereafter in the singular, is understood to mean, for the purpose of the present invention, one combustible gas or combustible mixture of several gases containing significant quantity of ethylene and having an overall flammable content over the upper flammability limit.

LVRG can be generated in at least one kind of units processing hydrocarbon sources in order to produce liquid hydrocarbon fractions. Such units could be hydrocarbon sources pyrolysis, hydro-pyrolysis, catalytic pyrolysis, electrical arc pyrolysis or Fischer-Tropsch synthesis. Hydrocarbon sources could be solid sources like coal, lignite and wood, liquid sources like oil (petroleum) and naphta or gaseous sources like synthesis gas. Such LVRG are usually burnt as fuel in the unit itself.

The expression "at least one kind of units processing hydrocarbon sources" is understood to mean, for the purpose of the present invention, that LVRG can be generated in one kind of units processing hydrocarbon sources or in several kinds of units processing hydrocarbon sources. Preferably, LVRG is generated in one kind of units processing hydrocarbon sources.

LVRG which is particularly preferred for the process according to the present invention is LVRG generated in oil refineries, usually called refinery off-gas (ROG) or a petrochemical off-gas.

The process according to the invention is therefore preferably a process starting from a refinery off-gas (ROG).

The expression "a refinery off-gas" (ROG), used hereafter in the singular, is understood to mean, for the purposes of the present invention, one combustible gas or a combustible mixture of several gases containing significant quantity of ethylene and having an overall flammable content over the upper flammability limit.

ROG can be generated in one or more of the units present in oil refineries. ROG are preferably generated in at least one of the following units present in oil refineries : fluid catalytic cracking (FCC), coker (delayed coker, fluid coker, flexicoker), gas plant, reformer, hydrocracker, hydrotreater and hydrodesulfuration (HDS). ROG is more preferably generated in at least one FCC unit.

ROG can be generated in one or in several oil refineries.

Most preferably, ROG is generated in one oil refinery and with a particular preference, in one FCC unit.

LVRG, preferably ROG, comprises advantageously from 3 to 60 % by weight of ethylene. LVRG, preferably ROG, comprises advantageously at least 3, preferably at least 5, more preferably at least 8 and most preferably at least 10 % by weight of ethylene. LVRG, preferably ROG, comprises advantageously at most 60, preferably at most 55, more preferably at most 50 and most preferably at most 48 % by weight of ethylene.

LVRG, preferably ROG, usually notably comprises :
- combustible gas like hydrogen, methane, ethane, ethylene, propane, propylene and hydrocarbons containing 4, 5 or 6 carbon atoms as well as heavier C6+ and hydrogen sulfide ;
- inert gases like nitrogen, carbon dioxide and water;
- at least one of the following oxidants : oxygen, carbon monoxide and nitrogen oxides;
- contaminants like hydrogen chloride, hydrogen cyanide, ammonia, nitrides, nitriles, carbonyl sulfide, organic compounds containing sulfur like sulfides and disulfides, sulfur oxides, acetylene, propadiene, methyl acetylene, butadiene, diethanolamine, methanol, phosphines, chlorides and organic compounds containing nitrogen; and
- traces of non carbon derivatives containing at least arsenic (like arsine), mercury, vanadium, bromine, fluorine, silicon, aluminium and metal carbonyls.

LVRG, preferably ROG, is characterized by a combustible gas content such that the lower heating value of the gas is advantageously comprised between 20 and 75 MJ/kg. LVRG, preferably ROG, is characterized by a combustible gas content such that the lower heating value of the gas is advantageously of at least 20, preferably of at least 25, more preferably of at least 30 and most preferably of at least 35 MJ/kg. LVRG, preferably ROG, is
characterized by a combustible gas content such that the lower heating value of the gas is advantageously of at most 75, preferably of at most 70, more preferably of at most 60 and most preferably of at most 55 MJ/kg.

LVRG, preferably ROG, comprises advantageously at most 25, preferably at most 20 and more preferably at most 15 % by volume of inert gases.

LVRG, preferably ROG, comprises oxidants in a total amount advantageously lower than the level needed to make the gaseous mixture flammable, preferably of at most 10, more preferably of at most 7 and most preferably of at most 5 % by volume.

LVRG, preferably ROG, comprises oxygen in an amount advantageously of at most 5, preferably of at most 3 and more preferably of at most 2 % by volume.

LVRG, preferably ROG, comprises carbon monoxide in an amount advantageously of at most 5, preferably of at most 3 and more preferably of at most 2 % by volume.

LVRG, preferably ROG, comprises contaminants in a total amount advantageously of at most 2000, preferably of at most 1500 and more preferably of at most 1000 ppm by volume.

LVRG, preferably ROG, comprises each contaminant in an individual amount advantageously of at most 500, preferably of at most 300 and more preferably of at most 200 ppm by volume.

LVRG, preferably ROG, comprises non carbon derivatives in a total amount advantageously of at most 5, preferably of at most 2 and more preferably of at most 1 ppm by volume.

LVRG, preferably ROG, comprises each non carbon derivative in an individual volume advantageously of at most 500, preferably of at most 300 and more preferably of at most 200 ppb by volume.

In the process for the manufacture of at least one ethylene derivative compound, in particular DCE, starting from a LVRG, preferably a ROG, according to the present invention, the LVRG, preferably the ROG, is subjected to a series of treatment steps in a LVRG, preferably ROG, recovery unit in order to remove the contaminants present therein and to obtain a mixture of products containing ethylene and other constituents (step a)).

When LVRG, preferably ROG, is a mixture of several gases, the different gases may be all subjected to the same series of treatment steps, each of them may be subjected to a dedicated series of treatment steps or each of them may be subjected to a combination of dedicated series of treatment steps and common series of treatment steps. Preferably each of them is subjected to a combination of dedicated series of treatment steps and common series of treatment steps.

The series of treatment steps in the LVRG, preferably ROG, recovery unit is advantageously composed of the following steps :
a1) optionally a compression step,
a2) oxygen removal
a2bis) nitrogen oxides removal
a2 ter) optionally non carbon derivatives removal
a3) hydrogenation,
a4) acid gases removal,
a5) cooling and drying,
a6) one or several adsorption steps
a7) optionally one or several absorption steps; and
a8) optionally a hydrogen, methane and/or nitrogen removal step.

A compression step (step a1)) is optionally performed.

When present, the compression step of the LVRG, preferably ROG, increases advantageously the pressure to at least 12, preferably to at least 14 kg/cm².g and advantageously to at most 50 kg/cm².g, preferably to at most 45 kg/cm².g.

The step a1) is preferably performed in several stages, either in a multistage gas compressor or in several compressors. A droplets separation is preferably performed before the compression step a1).

The compression ratio at each compression stage is such that the temperature at the exit of the compression stage is advantageously of at most 150, preferably of at most 120 and more preferably of at most 100°C. The gas which exits the stage is afterwards advantageously cooled down by indirect cooling with a cooling media. The cooling media is advantageously chosen among cooling tower water, cold water, atmospheric air and colder gas issued from the process. The cooling media is preferably chosen among cooling tower water and atmospheric air. The cooling fluid is more preferably cooling tower water.

The gas is advantageously cooled down under 50, preferably under 48 and more preferably under 45°C but advantageously not under 0, preferably not under 5 and more preferably not under 10°C.

The condensates can be separated or not. They are preferably separated. The condensates are advantageously degassed by pressure release, preferably pressure release at the pressure of the upstream stage. A stripping of the separated liquids can be performed in order to recover the volatile fractions. The produced gas is more preferably recycled with the gases of the upstream stage.

The bulk of the oxygen can be removed (step a2)) by a chemical step or by a physical step.

A suitable chemical step is advantageously performed by using a reduced bed of copper or a sulphided nickel catalyst, preferably by using a sulphided nickel catalyst.

Another suitable chemical step is advantageously a hydrogenation step which can be catalyzed or not, preferably is catalyzed.

The hydrogenation step may be performed by means of any known hydrogenation catalyst such as, for example, catalysts based on palladium, platinum, rhodium, ruthenium or iridium deposited on a support such as alumina, silica, silica/alumina, carbon, calcium carbonate or barium sulphate, but also catalysts based on nickel and those based on the cobalt-molybdenum complex. Preferably, the hydrogenation step is performed by means of a catalyst based on palladium or platinum deposited on alumina or carbon, on a catalyst based on nickel or on a catalyst based on the cobalt-molybdenum complex. In a particularly preferred manner, it is performed by means of a catalyst based on nickel.

The hydrogenation step uses advantageously part of the hydrogen available in the LVRG, preferably ROG.

A suitable physical process is advantageously performed by adsorption, absorption, via a PSA (pressure swing adsorption) or via a membrane process.

After step a2), the quantity or residual oxygen is advantageously of at most 2000, preferably of at most 1500, more preferably of at most 1000 and most preferably of at most 500 ppm by volume.

Step a2) is advantageously performed at a temperature between 25 and 100°C.

The bulk of the nitrogen oxides (step a2bis)) can be removed by a chemical step or by a physical step.

A suitable chemical step is advantageously performed by denox with ammonia or urea, preferably with urea.

Another suitable chemical step is advantageously a hydrogenation step which can be catalyzed or not, preferably catalyzed.

The hydrogenation step may be performed by means of the same catalysts as those defined for the hydrogenation of oxygen, with the same preferences. The hydrogenation step uses advantageously part of the hydrogen available in the LVRG, preferably ROG.

Hydrogenation is preferred over denox.

A suitable physical process is advantageously performed by adsorption, absorption, via a PSA (pressure swing adsorption) or via a membrane process.

After step a2bis), the quantity or residual nitrogen oxides is advantageously of at most 200, preferably of at most 150, more preferably of at most 100 and most preferably of at most 50 ppm by volume.

Step a2bis) is advantageously performed at a temperature between 25 and 100°C.

Non carbon derivatives are optionally removed (step a2ter)). They are advantageously removed by a physical process like adsorption on a suitable solid like active carbon, charcoal, molecular sieve or alumina.

The hydrogenation (step a3)) of undesirable derivatives such as, for example, acetylene, is advantageously performed in an acetylene converter by using a hydrogenation catalyst. Suitable catalytic species include advantageously metals of group VIII, metals of group Ib and metals of group VIIb. Catalysts based on palladium, on nickel or on gold are preferred. Catalysts based on palladium or on nickel are more preferred. Nickel based catalysts are most preferred with a particular preference for sulphided nickel catalysts. The hydrogenation catalysts may be supported or not. They are preferably supported. In other words, catalysts such as those defined for step a2) may be used.

Carbonyl sulfide, if still present in the hydrogenation feed, is advantageously converted into mercaptans during hydrogenation step 3), preferably with a palladium or nickel based catalyst, more preferably with a sulphided nickel catalyst.

Nitrogen oxides, if still present in the hydrogenation feed, are also advantageously converted into ammonia during hydrogenation step a3), preferably with a palladium or nickel based catalyst, more preferably with a sulphided nickel catalyst.

Nitriles present in the hydrogenation feed are also advantageously converted, preferably with a palladium or nickel based catalyst, more preferably with a sulphided nickel catalyst, into amines during hydrogenation step a3).

Arsine, if still present in the hydrogenation feed, is also advantageously removed during hydrogenation step a3), preferably with a palladium or nickel based catalyst, more preferably with a sulphided nickel catalyst.

Hydrogen cyanide and organic compounds containing nitrogen, if still present in the hydrogenation feed, are advantageously removed during hydrogenation step a3), preferably with a palladium or nickel based catalyst, more preferably with a sulphided nickel catalyst.

Higher acetylenic compounds present in the hydrogenation feed including methylacetylene, propadiene and butadiene are advantageously at least partially hydrogenated, preferably with a palladium or nickel based catalyst, more preferably with a sulphided nickel catalyst.

Step a3) is advantageously performed at a temperature between 25 and 100°C.

The acid gases removal (step a4)) may be performed in one or several steps. One step is advantageously an absorption with a regenerable solution like an amine, preferably alkanolamine, solution or with a physical sorbent like methanol. Another step is advantageously an absorption with chemical reaction performed by scrubbing in an alkaline solution. The alkali is preferably a hydroxide, an oxide or a carbonate. Examples of alkalis are sodium hydroxide, potassium hydroxide, calcium oxide, magnesium oxide, sodium carbonate and potassium carbonate.

The acid gases removal step a4) comprises preferably a first step which is an absorption with a regenerable solution of an amine, preferably alkanolamine, followed by an absorption with an alkaline solution (caustic/water wash tower), preferably sodium hydroxide solution.

The regenerable solution may be regenerated or not. If regeneration takes place, it occurs advantageously in one or several stages, in particular in order to separate carbon dioxide and hydrogen sulfide. The regenerable solution is preferably regenerated and more preferably in two stages.

The acid gases removal step a4) comprises more preferably a first step which is an absorption with a regenerable solution of an amine, preferably alkanolamine, which is regenerated in two stages, followed by an absorption with an alkaline solution (caustic/water wash tower), preferably sodium hydroxide solution.

The acid gases which are removed by such step a4) are advantageously hydrogen sulphide, hydrogen halides with in particular hydrogen chloride, carbonyl sulfide, hydrogen cyanide and carbon dioxide.

The organic compounds containing sulfur like sulfides and disulfides as well as sulfur oxides can be partially hydrolyzed during step a4). Diethanolamine and chlorides are advantageously removed during step a4). Methanol is advantageously removed with water during step a4).

The cooling and drying (step a5)) is advantageously performed by indirect cooling with a cooling media. The cooling media is advantageously chosen among cooling tower water, cold water, atmospheric air and colder gas issued from the process. The cooling media is preferably chosen among cooling tower water and atmospheric air. The cooling fluid is more preferably cooling tower water.

The gas is advantageously cooled down under 50, preferably under 48 and more preferably under 45°C but advantageously not under 0, preferably not under 5 and more preferably not under 10°C.

The condensates can be separated or not. They are preferably separated. The condensates are afterwards advantageously dried by adsorption, for example in a molecular sieve dryer or with silica gel or alumina, in order to remove water.

Alternatively, a freezed drying step can be used to dry.

Drying is preferably performed before cooling.

One or several adsorption steps (step a6)) are advantageously performed on chemically specific adsorbents, preferably arranged in a single mixed bed or in a series of adsorbent beds, in order to remove the other contaminants.

Mercaptans derived from carbonyl sulfide as well as carbonyl sulfide, are advantageously removed via adsorption in a bed of a suitable material. Suitable adsorbents include advantageously carboneous material such as activated carbon and particularly activated carbon having a specific surface between 500 and 2500 m2/g, molecular sieve 3, 4A or 13X, a zeolithe, a mesoporous adsorbent including activated alumina such as a mesoporous activated alumina with a specific BET surface between 150 m/g and 800 m/g, silica gel, a mesoporous silica gel adsorbent with a specific BET surface between 150 m/g and 800 m/g, a type A zeolithe, a type 5A zeolithe, a type X faujasite zeolithe, a type Y faujasite zeolithe and a MFI zeolithe. Preferred are activated carbon, molecular sieve 3 or 4A and activated alumina.

Ammonia is advantageously removed by adsorption with the same kind of adsorbents as for removing mercaptans, if not removed already by water wash.

Nitrogen oxides and carbon dioxide are advantageously removed by adsorption on a suitable adsorbent if not completely converted to ammonia in the hydrogenation step. Suitable adsorbents include activated copper, mineral clays, silica gel and activated alumina.

Amines derived from nitriles as well as residual nitriles, are advantageously removed via adsorption with the same kind of adsorbents as for removing mercaptans. Nitrides may also be partially adsorbed during step a6).

Mercury is advantageously removed by adsorption on an activated carbon bed. Arsine and phosphines may also be partially adsorbed during step a6).

Advantageously at least 1, preferably at least 2 adsorbents are used for the adsorption step a6). Advantageously at most 6, preferably at most 5, more preferably at most 4 adsorbents are used for the adsorption step a6). Most preferably 3 adsorbents are used.

The gas stream can contacted with the solid adsorbents in any suitable devices. Pneumatic conveying moving beds and fixed beds can be cited as suitable devices. Fixed beds are preferred.

The adsorbents can be arranged in mixed beds or in dedicated beds. They can be arranged in a single vessel or in separated vessels. The adsorbents are preferably arranged in dedicated beds, more preferably in 3 dedicated beds, and preferably in separated vessels.

Each adsorption step can be realized in one or several parallel beds. Each adsorption step is preferably realized in several parallel beds, more preferably in at least 2 separated beds.

Regeneration can be realized in the apparatus itself or outside the apparatus. Regeneration is preferably realized in the apparatus itself.

Step a6) is advantageously performed at a temperature between 25 and 100°C.

One or several absorption steps a7) are optionally performed. One or several physical absorption steps are advantageously performed with a suitable solvent, for example with dimethyletherpolyethylene glycol in order to remove among others, hydrogen sulphide, organic compounds containing sulfur like sulfides and disulfides, carbonyl sulfide, ammonia, hydrogen cyanide and metal carbonyls. One or several chemical absorption steps are advantageously performed with a suitable solvent in order to remove among others, arsine.

At least part of hydrogen, methane and/or nitrogen may be removed. This removal is optionally performed during step a) (step a8)) of the process according to the invention. Such step for remove at least part of hydrogen, methane and/or nitrogen may also be performed during step b) of the process according to the invention, for example during separation of the mixture of products derived from step a) or on fraction A. Preferably, when performed, removal of at least part of hydrogen, methane and/or nitrogen is performed during step a) of the process according to the invention.

Suitable separation steps for hydrogen, methane and/or nitrogen are membrane permeation and pressure swing adsorption (PSA). PSA is preferred.

The different steps mentioned before are not necessary performed in the order they are recited. They can be realized in any other order.

Step a5) is however advantageously the last step of the treatment steps.

A preferred order according to which the treatment steps take place is :
1. a1) with step a4) intercalated before the last compression stage,
2. steps a2) and a2bis) with optionally part of step a6),
3. step a6),
4. step a3),
5. optionally part of step a6),
5. step a5).

Step a7) can be at least partially included in step a4).

Steps a2)/a2bis) and step a6) can be mixed/integrated, optionally with step a2ter).

Step a3) can be inserted into step 6).

Advantageously, in the process according to the invention, the mixture of products containing ethylene and other constituents derived from step a) comprises hydrogen, nitrogen, carbon monoxide, methane, ethane, ethylene, propane, propylene, hydrocarbons containing 4, 5 or 6 carbon atoms and heaviers C6+.

The mixture of products containing ethylene and other constituents derived from step a) advantageously comprises at least 5, preferably at least10, more preferably at least 15 % by volume of ethylene. It advantageously comprises at most 60, preferably at most 55" more preferably at most 50 % by volume of ethylene.

The mixture of products containing ethylene and other constituents derived from step a) is advantageously characterized by a combustible gas content such that the lower heating value of the gas is advantageously of at least 30, preferably of at least 33, more preferably of at least 35 and most preferably of at least 37 MJ/kg. The mixture of products containing ethylene and other constituents derived from step a) is advantageously characterized by a combustible gas content such that the lower heating value of the gas is advantageously of at most 75, preferably of at most 70, more preferably of at most 65 and most preferably of at most 60 MJ/kg.

The mixture of products containing ethylene and other constituents derived from step a) advantageously comprises at most 28, preferably at most 23, more preferably at most 18 % by volume of nitrogen.

The mixture of products containing ethylene and other constituents derived from step a) advantageously comprises at most 5, preferably at most 3, more preferably at most 2 % by volume of carbon monoxide.

The dew point of water comprised in the mixture of products containing ethylene and other constituents derived from step a) is advantageously less than -50°C at atmospheric pressure.

The mixture of products containing ethylene and other constituents derived from step a) advantageously comprises at most 5 % of the LVRG, preferably ROG, content of oxygen, nitrogen oxides, acetylene, carbon dioxide, hydrogen sulfide, carbonyl sulfide, organic compounds containing sulfur like sulfides, sulfur oxides, diethanolamine, ammonia, hydrogen chloride, methanol, chlorides, hydrogen cyanide, mercury, vanadium, bromine, fluorine, silicon, aluminium and metal carbonyls.

The mixture of products containing ethylene and other constituents derived from step a) advantageously comprises at most 50 % of the LVRG, preferably ROG, content of non carbon derivatives containing at least arsenic (like arsine), phosphines, disulfides, propadiene, methyl acetylene, butadiene, nitrides, nitriles and organic compounds containing nitrogen.

After step a) defined above, the mixture of products containing ethylene and other constituents is subjected to step b) which advantageously comprises a maximum of four, preferably a maximum of three separation steps in order to obtain the two fractions containing ethylene, namely fraction A and fraction B.

According to the process according to the invention, fraction A and fraction B are conveyed to the manufacture of at least one ethylene derivative compounds.

In the more preferred process according to the invention for the manufacture of one ethylene derivative compound which is DCE, fraction A is conveyed to the chlorination reactor and fraction B to the oxychlorination reactor, preferably after expansion with recovery of energy.

According to the more preferred process of the invention, the quantities defined below to characterize the fraction B and the fraction A are those before their respective entry into oxychlorination and into chlorination.

Fraction B is advantageously characterized by a hydrogen content of less than or equal to 2 %, preferably of less than or equal to 0.5 % and in a particularly preferred manner of less than or equal to 0.1 % by volume relative to the total volume of fraction B.

Fraction B is characterized by a content of compounds containing at least 3 carbon atoms, advantageously less than or equal to 0.01 %, preferably less than or equal to 0.005 % and in a particularly preferred manner less than or equal to 0.001 % by volume relative to the total volume of fraction B.

Fraction B advantageously contains from 40 % to 99.5 % by volume of ethylene relative to the total volume of fraction B. Fraction B advantageously contains at least 40 %, preferably at least 50 % and in a particularly preferred manner at least 60 % by volume of ethylene relative to the total volume of fraction B. Fraction B advantageously contains at most 99.5 %, preferably at most 99.2 % and in a particularly preferred manner at most 99 % by volume of ethylene relative to the total volume of fraction B.

Fraction B is additionally characterized by an acetylene content which is advantageously less than or equal to 0.01 %, preferably less than or equal to 0.005 % and in a particularly preferred manner less than or equal to 0.001 % by volume relative to the total volume of fraction B.

Fraction A is enriched with compounds which are lighter than ethylene. These compounds are generally methane, nitrogen, oxygen, hydrogen and carbon monoxide. Advantageously, fraction A contains at least 70 %, preferably at least 80 % and in a particularly preferred manner at least 85 % of compounds lighter than ethylene which are contained in the mixture of products subjected to step b). Advantageously, fraction A contains at most 99.99 %, preferably at most 99.97 % and in a particularly preferred manner at most 99.95 % of compounds lighter than ethylene which are contained in the mixture of products subjected to step b).

Fraction A is characterized by a content of compounds containing at least 3 carbon atoms, advantageously less than or equal to 0.01 %, preferably less than or equal to 0.005 % and in a particularly preferred manner less than or equal to 0.001 % by volume relative to the total volume of fraction A.

Fraction A advantageously contains a content by volume of ethylene such that it represents from 10 % to 90 % of the content by volume of ethylene of fraction B. Fraction A advantageously contains a content by volume of ethylene such that it is less than or equal to 90 %, preferably less than or equal to 85 % and in a particularly preferred manner less than or equal to 80 % of the content by volume of ethylene of fraction B. Fraction A advantageously contains a content by volume of ethylene such that it is at least 10 %, preferably at least 15 % and in a particularly preferred manner at least 20 % of the content by volume of ethylene of fraction B.

Fraction A is additionally characterized by an acetylene content which is advantageously less than or equal to 0.01 %, preferably less than or equal to 0.005 % and in a particularly preferred manner less than or equal to 0.001 % by volume relative to the total volume of fraction A.

According to a first variant of the process according to the invention, considering that the process for the manufacture of DCE is advantageously balanced (that is to say that the process of manufacture by chlorination and oxychlorination of ethylene and pyrolysis of the 1,2-dichloroethane (DCE) formed makes it possible to generate the quantity of HCl necessary for the process), the fraction by weight of the ethylene throughput in each of fractions A and B is advantageously between 45 and 55 % of the total quantity of ethylene produced (fraction A + fraction B). Preferably, the fraction by weight of the throughput of ethylene in fraction A is of the order of 55 % and the fraction by weight of the throughput of ethylene in fraction B is of the order of 45 % of the total quantity produced. In a particularly preferred manner, the fraction by weight of the throughput of ethylene in fraction A is of the order of 52.5 % and the fraction by weight of the throughput of ethylene in fraction B is of the order of 47.5 % of the total quantity produced.

According to a second variant of the process according to the invention, considering that the process for the manufacture of DCE is advantageously unbalanced (that is to say for example that an external source of HCl makes it possible to provide part of the supply of HCl for the oxychlorination or that a fraction of the DCE produced is not subjected to pyrolysis), the fraction by weight of the throughput of ethylene in each of fractions A and B is advantageously between 20 and 80 % of the total quantity of ethylene produced (fraction A + fraction B). Preferably, the fraction by weight of the throughput of ethylene in fraction A is between 25 and 75 % of the total quantity of ethylene produced (fraction A + fraction B).

According to a first embodiment of the second variant of the process according to the invention, considering that the process for the manufacture of DCE is advantageously unbalanced by an external source of HCl, the fraction by mole of the throughput of ethylene in fraction A is advantageously between 45 and 55 %, preferably between 50 and 54 % and in a particularly preferred manner of the order of 52.5 % of the difference between the total molar quantity of ethylene contained in the mixture of products subjected to step b) and the molar quantity of HCl of the external source.

According to a second embodiment of the second variant of the process according to the invention, considering that the process for the manufacture of DCE is advantageously unbalanced by a co-production of DCE (some of the DCE is therefore not subjected to pyrolysis), the fraction by mole of the throughput of ethylene in fraction B is advantageously between 45 and 55 %, preferably between 46 and 50 % and in a particularly preferred manner of the order of 47.5 % of the difference between the total molar quantity of ethylene contained in the mixture of products subjected to step b) and the molar quantity of DCE co-produced.

According to the process of the invention, during step b), the mixture of products is separated into a fraction enriched with the compounds lighter than ethylene containing some of the ethylene (fraction A), into a fraction enriched with ethylene (fraction B) and into a heavy fraction (fraction C).

Fraction C advantageously contains ethane and compounds comprising at least 3 carbon atoms. Advantageously, these compounds comprising at least 3 carbon atoms result from the mixture of products containing ethylene and other constituents derived from step a) or are generated by side reactions during step b). Among the compounds comprising at least 3 carbon atoms, there may be mentioned propane, propylene, butanes and their unsaturated derivatives as well as all the saturated or unsaturated heavier compounds.

Fraction C contains advantageously at least 80, preferably at least 85 and particularly preferably at least 90 % of the ethane contained in the mixture of products subjected to step b).

Fraction C advantageously contains at least 95 %, preferably at least 98 % and particularly preferably at least 99 % of compounds comprising at least 3 carbon atoms contained in the mixture of products subjected to step b).

Fraction C advantageously contains at most 1 %, preferably at most 0.8 % and particularly preferably at most 0.5 % by weight of ethylene relative to the total weight of fraction C.

Fraction C is advantageously enriched in components heavier than ethylene. Preferably, fraction C is burnt or valorised chemically. More preferably, fraction C is valorised chemically.

According to a first embodiment of the process according to the invention, the mixture of products derived from step a) is advantageously subjected to a first separation step called step S1 and to a second separation step called step S1' in order to obtain the two fractions containing ethylene, namely fraction A and fraction B.

Step S1 advantageously consists in the separation of the mixture of products derived from step a) inside a main column (called column C1) into three different fractions, namely fraction A which leaves at the top of column C1, fraction C which leaves at the bottom of column C1 and a fraction (called fraction F1) which is drawn off from the side of column C1.

Step S1' advantageously consists in separating fraction F1 into two different fractions, namely a fraction F1' which is conveyed to the column C1 and fraction B.

According to the first embodiment of the process according to the invention, step b) therefore preferably comprises :
- a first separation step S1 which consists in the separation of the said mixture of products inside a main column C1 into fraction A at the top of column C1, into fraction C at the bottom of column C1 and into fraction F1 drawn off from the side of column C1, and
- a second separation step S1' which consists in the separation of fraction F1 into a fraction F1' which is conveyed to the column C1 and into fraction B.

In a particularly preferred manner, step b) comprises only the two steps mentioned above.

Prior to its introduction into column C1, the mixture of products derived from step a) may be subjected to a heat conditioning step. The expression heat conditioning step is understood to mean a succession of heat exchanges optimizing the use of energy, for example the gradual cooling of the mixture of products in a train of exchangers first cooled with untreated water, and then with ice-cold water and then with increasingly cooled fluids plus cross exchangers recovering the sensible heat of the streams produced.

The said mixture of products may be introduced into the column C1 during step S1 as a single fraction or as several subfractions. It is preferably introduced as several subfractions.

The main column C1 is advantageously a column comprising a stripping section and/or a rectifying section. If the two sections are present, the rectifying section preferably surmounts the stripping section.

The column C1 is advantageously chosen from distillation columns comprising the abovementioned two sections and the columns containing only one of the two sections. Preferably, the column C1 is a distillation column.

Step S1 is therefore preferably a distillation step.

The column C1 is advantageously provided with the associated auxiliary equipment such as for example at least one reboiler and at least one condenser. Devices allowing intermediate drawing off and an intermediate heat exchange may be added to the main column.

Fraction A enriched with the most volatile compounds advantageously leaves at the top of column C1 whereas fraction C enriched with the least volatile compounds advantageously leaves at the bottom of column C1.

As for fraction F1, it is advantageously drawn off from the side of the column C1 by collecting liquid or steam circulating in the column. The drawing off is preferably performed on the liquid.

The drawing off may be performed in the stripping section or in the rectifying section of the column. It is preferably performed in the rectifying section. A drawing off in the central third of the rectifying section is particularly preferred. The drawing off of liquid in the central third of the rectifying section is most particularly preferred.

The abovementioned step S1 is advantageously performed at a pressure of at least 15, preferably of at least 20 and in a particularly preferred manner of at least 25 bar. Step S1 is advantageously performed at a pressure of at most 45, preferably of at most 40 and in a particularly preferred manner of at most 38 bar.

The temperature at which step S1 is performed is advantageously at least -70, preferably at least -65 and in a particularly preferred manner at least -60°C at the top of column C1. It is advantageously at most -30, preferably at most -40 and in a particularly preferred manner at most -50°C at the top of column C1.

The fraction F1 drawn off from the side of the column C1 is advantageously subjected to the separation step S1' so as to be separated into two different fractions, namely a fraction F1' which is conveyed to the column C1 and fraction B.

Fraction F1 may be drawn off from the column C1 in the liquid state or in the gaseous state.

If the fraction F1 is drawn off in the liquid state, it may be conveyed to an evaporator or to an auxiliary column C1'.

In the case where the fraction F1 is conveyed to an evaporator, part of fraction F1, in the form of a fraction F1', is advantageously evaporated and recycled to the main column C1 while the other part is advantageously extracted from the evaporator thus constituting fraction B. As a variant, fraction F1 may also be partially vaporized in order to produce fraction B, the balance, in the form of a fraction F1', being recycled to the column C1.

In the case where the fraction F1 is conveyed to an auxiliary column C1', the auxiliary column C1' is preferably a stripping column, namely a column which comprises only one stripping section. The auxiliary column C1' is advantageously provided with associated auxiliary equipment, preferably a reboiler. Fraction B is advantageously extracted therefrom and the balance of fraction F1, in the form of a fraction F1' which is then a stream concentrated with impurities more volatile than ethylene (H₂, CO, N₂, O₂ and CH₄), is advantageously conveyed to the column C1.

If the fraction F1 is drawn off in the liquid state, it is preferably conveyed to an auxiliary column C1' which is preferably a stripping column. Step S1' is then in this case preferably a stripping step.

If the fraction F1 is drawn off in the gaseous state, it may be conveyed to a condenser or to an auxiliary column C1'.

In the case where the fraction F1 is conveyed to a condenser, part of fraction F1, in the form of a fraction F1', is advantageously condensed and recycled to the main column C1 while the other part is advantageously extracted from the condenser thus constituting the fraction B. As a variant, the fraction F1 may also be partially condensed in order to produce the fraction B, the balance, in the form of a fraction F1', being recycled to the column C1.

In the case where the fraction F1 is conveyed to an auxiliary column C1', the auxiliary column C1' is preferably a rectifying column, namely a column which comprises only a rectifying section. The auxiliary column C1' is advantageously provided with associated auxiliary equipment, preferably a condenser. The fraction B is advantageously extracted therefrom and the balance of the fraction F1 in the form of a fraction F1' which is then a stream concentrated with impurities less volatile than ethylene (ethane, compounds containing at least 3 carbon atoms), is advantageously conveyed to the column C1.

If the fraction F1 is drawn off in the gaseous state, it is preferably conveyed to an auxiliary column C1' which is preferably a rectifying column. Step S1' is then in this case preferably a rectifying step.

According to the first embodiment of the process according to the invention, a most particular preference is given to the case where the fraction F1 is conveyed to an auxiliary column C1'.

According to this most particular preference, step b) therefore comprises in a particularly preferred manner :
- a first separation step S1 which consists in the separation of the said mixture of products inside a main column C1 into fraction A at the top of column C1, into fraction C at the bottom of column C1 and into fraction F1 drawn off from the side of column C1, and
- a second separation step S1' which consists in the separation of fraction F1 inside a column C1' into a fraction F1' at the top of column C1' which is conveyed to the column C1 and into fraction B at the bottom of column C1'.

According to the first embodiment of the process according to the invention, a truly most particular preference is given to the case where the fraction F1 is drawn off from the column C1 in the liquid state and conveyed to an auxiliary column C1' which is a stripping column.

The abovementioned step S1' is then advantageously performed at a pressure of at least 15, preferably of at least 25 and in a particularly preferred manner of at least 30 bar. Step S1' is advantageously performed at a pressure of at most 45, preferably of at most 40 and in a particularly preferred manner of at most 38 bar.

The temperature at which step S1' is performed is advantageously at least -40, preferably at least -30 and in a particularly preferred manner at least -25°C at the top of the stripping column C1'. It is advantageously at most 0, preferably at most -10 and in a particularly preferred manner at most -15°C at the top of column C1'.

The temperature at the bottom of the stripping column C1' is at least -30, preferably at least -20 and in a particularly preferred manner at least -15°C. It is advantageously at most 20, preferably at most 15 and in a particularly preferred manner at most 10°C.

According to the first embodiment of the process according to the invention, fraction B is advantageously conveyed to the oxychlorination reactor, preferably after evaporation and expansion if fraction F1 is drawn off in the liquid state or after expansion if fraction F1 is drawn off in the gaseous state, in both cases advantageously with energy recovery. In a particularly preferred manner, fraction B is conveyed to the oxychlorination reactor after evaporation and expansion in the case where fraction F1 is drawn off in the liquid state, advantageously with energy recovery.

A preferred subvariant of the first embodiment of the process according to the invention is to carry out the separation step S1' by means of an auxiliary column C1' identical to the main column C1, both columns being optionally thermally integrated and operating at different pressures; the condenser of one serving as the reboiler to the other.

According to a second embodiment of the process according to the invention, the mixture of products derived from step a) is advantageously subjected to a first separation step called step S2, to a second separation step called step S2' and to a third separation step called step S2" in order to obtain the two fractions containing ethylene, namely fraction A and fraction B.

Step S2 advantageously consists in the separation of the mixture of products derived from step a) in a main column (called column C2) into two different fractions, namely a fraction F2 which leaves at the top of column C2 and fraction C which leaves at the bottom of column C2.

Step S2' advantageously consists in the separation of fraction F2 into two different fractions, namely fraction A and a fraction F2'.

Step S2'' advantageously consists in the separation of fraction F2' into two different fractions, namely fraction B and a fraction F2''.

According to the second embodiment of the process according to the invention, step b) therefore preferably comprises :
- a first separation step S2 which consists in the separation of the said mixture of products in a main column C2 into a fraction F2 at the top of column C2 and into fraction C at the bottom of column C2,
- a second separation step S2' which consists in the separation of fraction F2 into fraction A and into a fraction F2', and
- a third separation step S2" which consists in the separation of fraction F2' into fraction B and into a fraction F2''.

In a particularly preferred manner, step b) comprises only the three steps mentioned above.

Prior to its introduction into the column C2, the mixture of products derived from step a) may be subjected to a heat conditioning step. The expression heat conditioning step is understood to mean a succession of heat exchanges optimizing the use of energy, for example the gradual cooling of the mixture of products in a train of exchangers first cooled with untreated water, then with ice-cold water and then with increasingly cold fluids plus cross exchangers recovering the sensible heat of the streams produced.

The said mixture of products may be introduced into the column C2 during step S2 as a single fraction or as several subfractions. It is preferably introduced as several subfractions.

The main column C2 is advantageously a column comprising a stripping section and/or a rectifying section. If the two sections are present, the rectifying section preferably surmounts the stripping section.

The column C2 is advantageously chosen from distillation columns comprising the abovementioned two sections and columns comprising only one of the two sections. Preferably, the column C2 is a distillation column.

Step S2 is therefore preferably a distillation step. The column C2 is advantageously provided with the associated auxiliary equipment such as for example at least one reboiler and at least one condenser.

The fraction F2 enriched with the most volatile compounds advantageously leaves at the top of column C2 while the fraction C enriched with the least volatile compounds advantageously leaves at the bottom of column C2.

The abovementioned step S2 is advantageously performed at a pressure of at least 15, preferably of at least 20 and in a particularly preferred manner of at least 25 bar. Step S2 is advantageously performed at a pressure of at most 45, preferably of at most 40 and in a particularly preferred manner of at most 38 bar.

The temperature at which step S2 is performed is advantageously at least -70, preferably at least -65 and in a particularly preferred manner at least -60°C at the top of column C2. It is advantageously at most -20, preferably at most -30 and in a particularly preferred manner at most -40°C at the top of column C2.

The fraction F2 which leaves at the top of column C2 is advantageously subjected to the separation step S2'so as to be separated into two different fractions, namely fraction A and a fraction F2'.

The separation step S2' is advantageously an absorption step in which fraction F2 is brought into contact with a washing agent containing DCE.

In the present description, the term "washing agent containing DCE" or more simply "washing agent" is understood to mean a composition in which the DCE is present in the liquid state.

The washing agent which may be used according to the present invention therefore advantageously contains DCE in the liquid state. The presence, in the said washing agent, of other compounds is not at all excluded from the scope of the invention. It is preferable, however, that the washing agent contains at least 50 % by volume of DCE, more particularly at least 80 % by volume and in a particularly preferred manner at least 95 % by volume.

The washing agent used for step S2' may consist of fresh washing agent of any origin, for example crude DCE leaving the oxychlorination unit and which has not been purified, the said DCE previously purified or washing agent recovered during step S2" detailed below (fraction F2"), optionally supplemented with fresh washing agent.

Preferably, the washing agent used for step S2' consists of the fraction F2", optionally supplemented with fresh washing agent. In a particularly preferred manner, the washing agent used for step S2' consists of the fraction F2" supplemented with fresh washing agent (to compensate for the loss of washing agent during steps S2' and S2").

A major advantage of the second embodiment of the process according to the invention lies in the fact that the presence of this DCE is not at all troublesome since it is the compound mainly formed during the oxychlorination or chlorination.

The ratio between the respective throughputs of washing agent and ethylene to be extracted from the fraction F2 is not critical and can vary to a large extent. It is in practice limited only by the cost of the regeneration of the washing agent. In general, the throughput of washing agent is at least 1, preferably at least 5 and in a particularly preferred manner at least 10 tons per ton of ethylene to be extracted from the fraction F2. In general, the throughput of washing agent is at most 100, preferably at most 50 and in a particularly preferred manner at most 25 tons per ton of ethylene to be extracted from the fraction F2.

Step S2' is advantageously performed by means of an absorber such as for example a falling or rising film absorber or an absorption column C2' chosen from plate columns, packed columns, columns with structured packing, columns combining one or more of the abovementioned internals and spray columns. Step S2' is preferably performed by means of an absorption column C2' and in a particularly preferred manner by means of a plate absorption column C2'.

The column C2' is advantageously provided with associated auxiliary equipment such as, for example, at least one condenser or one cooler internal or external to the column.

The abovementioned step S2' is advantageously performed at a pressure of at least 15, preferably of at least 20 and in a particularly preferred manner at least 25 bar. Step S2' is advantageously performed at a pressure of at most 40, preferably at most 35 and in a particularly preferred manner at most 30 bar.

The temperature at which step S2' is performed is advantageously at least -10, preferably at least 0 and in a particularly preferred manner at least 10°C at the top of the absorber or of column C2'. It is advantageously at most 60, preferably at most 50 and in a particularly preferred manner at most 40°C at the top of the absorber or column C2'.

The temperature at the bottom of the absorber or column C2' is at least 0, preferably at least 10 and in a particularly preferred manner at least 20°C. It is advantageously at most 70, preferably at most 60 and in a particularly preferred manner at most 50°C.

The fraction F2' is advantageously subjected to the separation step S2'' so as to be separated into two different fractions, namely fraction B and a fraction F2''.

The separation step S2" is advantageously a desorption step in which fraction B is extracted from the washing agent.

The washing agent recovered after step S2'' constituting the fraction F2" may be removed, conveyed completely or partly to the oxychlorination section or conveyed to step S2' with optional addition of fresh washing agent. Preferably, the fraction F2" is conveyed to step S2' with optional addition of fresh washing agent. In a particularly preferred manner, the fraction F2" is conveyed to step S2' with addition of fresh washing agent.

Step S2'' is advantageously performed by means of a desorber such as for example a falling or rising film desorber, a reboiler or a desorption column C2" chosen from plate columns, packed columns, columns with structured packing, columns combining one or more of the abovementioned internals and spray columns. Step S2'' is preferably performed by means of a desorption column C2" and in a particularly preferred manner by means of a plate desorption column C2''.

The column C2" is advantageously provided with associated auxiliary equipment such as for example at least one condenser or one cooler internal or external to the column and at least one reboiler.

The abovementioned step S2'' is advantageously performed at a pressure of at least 1, preferably of at least 2 and in a particularly preferred manner of at least 3 bar. Step S2'' is advantageously performed at a pressure of at most 20, preferably of at most 15 and in a particularly preferred manner of at most 10 bar.

The temperature at which step S2'' is performed is advantageously chosen so that more than 90 %, preferably more than 95 % of the ethylene contained in the fraction F2' is found in fraction B. The temperature at which step S2" is performed is advantageously at least -10, preferably at least 0 and in a particularly preferred manner at least 10°C at the top of the desorber or of column C2''. It is advantageously at most 60, preferably at most 50 and in a particularly preferred manner at most 40°C at the top of the desorber or column C2''.

The temperature at the bottom of the desorber or column C2" is at least 60, preferably at least 80 and in a particularly preferred manner at least 100°C. It is advantageously at most 200, preferably at most 160 and in a particularly preferred manner at most 150°C.

According to the second embodiment of the process according to the invention, a most particular preference is given to the case where the fraction F2 is conveyed to an absorption column C2' and the fraction F2' is conveyed to a desorption column C2''.

According to this most particular preference, step b) therefore comprises in a particularly preferred manner :
- a first separation step S2 which consists in the separation of the said mixture of products in a main column C2 into a fraction F2 at the top of column C2 and into fraction C at the bottom of column C2,
- a second separation step S2' which consists in the separation of the fraction F2 in an absorption column C2' into fraction A at the top of column C2' and into a fraction F2' at the bottom of column C2', and
- a third separation step S2" which consists in the separation of the fraction F2' in a desorption column C2" into fraction B at the top of column C2" and into a fraction F2" at the bottom of column C2''.

According to a third embodiment of the process according to the invention, the mixture of products derived from step a) is advantageously subjected to a first separation step called step S3 and to a second separation step called step S3' in order to obtain the two fractions containing ethylene, namely fraction A and fraction B.

Step S3 advantageously consists in the separation of the mixture of products derived from step a) in a main column (called column C3) into two different fractions, namely a fraction F3 which leaves at the top of column C3 and the fraction C which leaves at the bottom of column C3.

Step S3' advantageously consists in the separation of the fraction F3 in a column C3' into two different fractions, namely the fraction A which leaves at the top of column C3' and the fraction B which leaves at the bottom of column C3'.

According to the third embodiment of the process according to the invention, step b) therefore preferably comprises :
- a first separation step S3 which consists in the separation of the said mixture of products in a main column C3 into a fraction F3 at the top of column C3 and into fraction C at the bottom of column C3, and
- a second separation step S3' which consists in the separation of the fraction F3 in a column C3' into fraction A at the top of column C3' and into fraction B at the bottom of column C3'.

In a particularly preferred manner, step b) comprises only the two steps mentioned above.

Prior to its introduction into the column C3, the mixture of products derived from step a) may be subjected to a heat conditioning step. The expression heat conditioning step is understood to mean a succession of heat exchanges optimizing the use of energy, for example the gradual cooling of the mixture of products in a train of exchangers first cooled with untreated water, and then with ice cold water and then with increasingly cold fluids plus cross exchangers recovering the sensible heat of the streams produced.

The said mixture of products may be introduced into the column C3 during step S3 as a single fraction or as several subfractions. It is preferably introduced as several subfractions.

The main column C3 is advantageously a column comprising a stripping section and/or a rectifying section. If the two sections are present, the rectifying section preferably surmounts the stripping section.

The column C3 is advantageously chosen from distillation columns comprising the abovementioned two sections and columns containing only one of the two sections. Preferably, the column C3 is a distillation column.

Step S3 is therefore preferably a distillation step. The column C3 is advantageously provided with the associated auxiliary equipment such as, for example, at least one reboiler and at least one condenser.

The fraction F3 enriched with the most volatile compounds advantageously leaves at the top of column C3 while the fraction C enriched with the least volatile compounds advantageously leaves at the bottom of column C3.

The abovementioned step S3 is advantageously performed at a pressure of at least 15, preferably of at least 20 and in a particularly preferred manner of at least 25 bar. The step S3 is advantageously performed at a pressure of at most 45, preferably of at most 40 and in a particularly preferred manner of at most 38 bar.

The temperature at which step S3 is performed is advantageously at least -70, preferably at least -65 and in a particularly preferred manner at least -60°C at the top of column C3. It is advantageously at most -20, preferably at most -30 and in a particularly preferred manner at most -40°C at the top of column C3.

The fraction F3 which leaves at the top of column C3 is then advantageously subjected to the separation step S3' in the column C3' so as to be separated into two different fractions, namely fraction A at the top of column C3' and fraction B at the bottom of column C3'.

The column C3' is advantageously a column comprising a stripping section and/or a rectifying section. If the two sections are present, the rectifying section preferably surmounts the stripping section.

The column C3' is advantageously chosen from the distillation columns comprising the abovementioned two sections and the columns comprising only one of the two sections. Preferably, the column C3' is a distillation column.

The step S3' is therefore preferably a distillation step.

The column C3' is advantageously provided with the associated auxiliary equipment such as, for example, at least one reboiler and at least one condenser.

The abovementioned step S3' is advantageously performed at a pressure of at least 15, preferably of at least 20 and in a particularly preferred manner of at least 25 bar. The step S3' is advantageously performed at a pressure of at most 40, preferably of at most 37 and in a particularly preferred manner of at most 35 bar.

The temperature at which the step S3' is performed is advantageously at least -70, preferably at least -67 and in a particularly preferred manner at least -65°C at the top of column C3'. It is advantageously at most -40, preferably at most -45 and in a particularly preferred manner at most -50°C at the top of column C3'.

The temperature at the bottom of column C3' is at least -30, preferably at least -25 and in a particularly preferred manner at least -20°C. It is advantageously at most 20, preferably at most 15 and in a particularly preferred manner at most 10°C.

According to a fourth embodiment of the process according to the invention, the mixture of products derived from step a) is advantageously subjected to a first separation step called step S4 and to a second separation step called step S4' in order to obtain the two fractions containing ethylene, namely fraction A and fraction B.

Step S4 advantageously consists in the separation of the mixture of products derived from step a) in a main column (called column C4) into two different fractions, namely fraction A which leaves at the top of column C4 and a fraction F4 which leaves at the bottom of column C4.

Step S4' advantageously consists in the separation of the fraction F4 in a column C4' into two different fractions, namely fraction B which leaves at the top of column C4' and fraction C which leaves at the bottom of column C4'.

According to the fourth embodiment of the process according to the invention, step b) therefore preferably comprises :
- a first separation step S4 which consists in the separation of the said mixture of products in a main column C4 into fraction A at the top of column C4 and into a fraction F4 at the bottom of column C4, and
- a second separation step S4' which consists in the separation of the fraction F4 in a column C4' into fraction B at the top of column C4' and into fraction C at the bottom of column C4'.

In a particularly preferred manner, step b) comprises only the two steps mentioned above.

Prior to its introduction into the column C4, the mixture of products derived from step a) may be subjected to a heat conditioning step. The expression heat conditioning step is understood to mean a succession of heat exchanges optimizing the use of energy, for example the gradual cooling of the mixture of products in a train of exchangers first cooled with untreated water, then with ice-cold water and then with increasingly cold fluids plus cross exchangers recovering the sensible heat of the streams produced.

The said mixture of products may be introduced into the column C4 during step S4 as a single fraction or as several subfractions. It is preferably introduced as several subfractions.

The main column C4 is advantageously a column comprising a stripping section and/or a rectifying section. If the two sections are present, the rectifying section preferably surmounts the stripping section.

The column C4 is advantageously chosen from the distillation columns comprising the abovementioned two sections and the columns comprising only one of the two sections. Preferably, the column C4 is a distillation column.

The step S4 is therefore preferably a distillation step. The column C4 is advantageously provided with the associated auxiliary equipment such as, for example, at least one reboiler and at least one condenser.

The fraction A enriched with the most volatile compounds advantageously leaves at the top of column C4 while the fraction F4 enriched with the least volatile compounds advantageously leaves at the bottom of column C4.

The abovementioned step S4 is advantageously performed at a pressure of at least 15, preferably of at least 20 and in a particularly preferred manner of at least 25 bar. The step S4 is advantageously performed at a pressure of at most 45, preferably of at most 40 and in a particularly preferred manner of at most 38 bar.

The temperature at which the step S4 is performed is advantageously at least -70, preferably at least -65 and in a particularly preferred manner at least -60°C at the top of column C4. It is advantageously at most -20, preferably at most -30 and in a particularly preferred manner at most -40°C at the top of column C4.

The fraction F4 which leaves at the bottom of column C4 is then advantageously subjected to the separation step S4' in the column C4' so as to be separated into two different fractions, namely the fraction B at the top of column C4' and the fraction C at the bottom of column C4'.

The column C4' is advantageously a column comprising a stripping section and/or a rectifying section. If the two sections are present, the rectifying section preferably surmounts the stripping section.

The column C4' is advantageously chosen from the distillation columns comprising the abovementioned two sections and the columns comprising only one of the two sections. Preferably, the column C4' is a distillation column.

The step S4' is therefore preferably a distillation step. The column C4' is advantageously provided with the associated auxiliary equipment such as, for example, at least one reboiler and at least one condenser.

The abovementioned step S4' is advantageously performed at a pressure of at least 15, preferably of at least 20 and in a particularly preferred manner of at least 25 bar. The step S4' is advantageously performed at a pressure of at most 40, preferably of at most 37 and in a particularly preferred manner of at most 35 bar.

The temperature at which the step S4' is performed is advantageously at least -50, preferably at least -40 and in a particularly preferred manner at least -30°C at the top of column C4'. It is advantageously at most 0, preferably at most -5 and in a particularly preferred manner at most -10°C at the top of column C4'.

The temperature at the bottom of column C4' is at least -20, preferably at least -15 and in a particularly preferred manner at least -10°C. It is advantageously at most 20, preferably at most 15 and in a particularly preferred manner at most 10°C.

According to a fifth embodiment of the process according to the invention, the mixture of products derived from step a) is advantageously subjected to a first separation step called step S5, to a second separation step called step S5' and to a third separation step called step S5" in order to obtain the two fractions containing ethylene, namely fraction A and fraction B.

Step S5 advantageously consists in the separation of the mixture of products derived from step a) in a main column (called column C5) into two different fractions, namely fraction A which leaves at the top of column C5 and a and a fraction F5 which leaves at the bottom of column C5.

Step S5' advantageously consists in the separation of the fraction F5 in a column C5' into two different fractions, namely a fraction F5' which leaves at the top of column C5' and fraction C which leaves at the bottom of column C5'.

Step S5" advantageously consists in the separation of the fraction F5' in a column C5" into two different fractions, namely fraction B which leaves at the top of column C5" and fraction F5" which leaves at the bottom of column C5".

According to the fifth embodiment of the process according to the invention, step b) therefore preferably comprises :
- a first separation step S5 which consists in the separation of the said mixture of products in a main column C5 into fraction A at the top of column C5 and into a fraction F5 at the bottom of column C5,
- a second separation step S5' which consists in the separation of the fraction F5 in a column C5' into fraction C at the top of column C5' and into a fraction F5' at the bottom of column C5'; and
- a third separation step S5" which consists in the separation of the fraction F5' in a column C5" into fraction B at the top of column C5" and into fraction F5" at the bottom of column C5"

In a particularly preferred manner, step b) comprises only the three steps mentioned above.

Prior to its introduction into the column C5, the mixture of products derived from step a) may be subjected to a heat conditioning step. The expression heat conditioning step is understood to mean a succession of heat exchanges optimizing the use of energy, for example the gradual cooling of the mixture of products in a train of exchangers first cooled with untreated water, then with ice-cold water and then with increasingly cold fluids plus cross exchangers recovering the sensible heat of the streams produced.

The said mixture of products may be introduced into the column C5 during step S5 as a single fraction or as several subfractions. It is preferably introduced as several subfractions.

The main column C5 is advantageously a column comprising a stripping section and/or a rectifying section. If the two sections are present, the rectifying section preferably surmounts the stripping section.

Column C5 is advantageously chosen from the distillation columns comprising the abovementioned two sections and the columns comprising only one of the two sections. Preferably, the column C5 is a distillation column.

The step S5 is therefore preferably a distillation step. The column C5 is advantageously provided with the associated auxiliary equipment such as, for example, at least one reboiler and at least one condenser.

Fraction A enriched with the compounds that are lighter than ethylene containing some of the ethylene, advantageously exits from the top of column C5 whereas fraction F5, advantageously enriched with the least volatile compounds, advantageously exits from the bottom of column C5.

The abovementioned step S5 is advantageously carried out at a pressure of at least 5, preferably at least 10 and particularly preferably at least 12 bar absolute. Step S5 is advantageously carried out at a pressure of at most 40, preferably at most 38 and particularly preferably at most 36 bar absolute.

The temperature at which step S5 is carried out is advantageously at least 0, preferably at least 5 and particularly preferably at least 10°C at the bottom of column C5. It is advantageously at most 80, preferably at most 60 and particularly preferably at most 40°C at the bottom of column C5.

The temperature at which step S5 is carried out is advantageously at least -70, preferably at least -60 and particularly preferably at least -55°C at the top of column C5. It is advantageously at most 0, preferably at most -15 and particularly preferably at most -25°C at the top of column C5.

The fraction F5 which leaves at the bottom of column C5 is then advantageously subjected to a second separation step S5' which consists of separating fraction F5 inside a column C5' into a fraction F5' and into a heavy fraction (fraction C).

Prior to its introduction into column C5', the mixture of products may be subjected to a thermal and/or chemical conditioning step, such as, for example, an acetylene hydrogenation. The term "thermal conditioning step" is understood to mean a series of heat exchanges optimizing the use of energy, for example the gradual cooling of the mixture of products in a set of exchangers first cooled with untreated water, then with iced water, and then with increasingly cold liquids plus cross exchangers recovering the sensible heat of the streams produced.

Said mixture of products may be introduced into column C5' during step S5' as a single fraction or as several subfractions. It is preferably introduced as several subfractions.

Column C5' is advantageously a column comprising a stripping section and/or a rectifying section. If both sections are present, the rectifying section preferably surmounts the stripping section.

Column C5' is advantageously chosen from distillation columns comprising the two aforementioned sections and the columns that only include one of the two sections. Preferably, column C5' is a distillation column.

Step S5' is therefore preferably a distillation step. Column C5' is advantageously equipped with associated accessories such as, for example, at least one reboiler and at least one condenser.

Fraction F5', advantageously enriched with the most volatile compounds, advantageously exits from the top of column C5' whereas the heavy fraction C, advantageously enriched with the least volatile compounds, advantageously exits from the bottom of column C5'.

The abovementioned step S5' is advantageously carried out at a pressure of at least 5, preferably at least 8 and particularly preferably at least 10 bar absolute. Step S5' is advantageously carried out at a pressure of at most 40, preferably at most 37 and particularly preferably at most 35 bar absolute.

The temperature at which step S5' is carried out is advantageously at least 0, preferably at least 10 and particularly preferably at least 15°C at the bottom of column C5'. It is advantageously at most 90, preferably at most 86 and particularly preferably at most 83°C at the bottom of column C5'.

The temperature at which step S5' is carried out is advantageously at least -65, preferably at least -55 and particularly preferably at least -50°C at the top of column C5'. It is advantageously at most 5, preferably at most 0 and particularly preferably at most -2°C at the top of column C5'.

The fraction F5 which leaves at the bottom of column C5 is then advantageously subjected to a second separation step S5' which consists of separating fraction F5 inside a column C5' into a fraction F5' and into a heavy fraction (fraction C).

The fraction F5' is subjected to a third separation step S5" which consists of separating fraction F5' inside a column C5" into a fraction enriched with ethylene (fraction B) and into a fraction F5" mainly composed of ethane.

Prior to its introduction into column C5", the mixture of products may be subjected to a thermal and/or chemical conditioning step, such as, for example, an acetylene hydrogenation. The term "thermal conditioning step" is understood to mean a series of heat exchanges optimizing the use of energy, for example the gradual cooling of the mixture of products in a set of exchangers first cooled with untreated water, then with iced water, and then with increasingly cold liquids plus cross exchangers recovering the sensible heat of the streams produced.

Said mixture of products may be introduced into column C5" during step S5" as a single fraction or as several subfractions. It is preferably introduced as several subfractions.

Column C5" is advantageously a column comprising a stripping section and/or a rectifying section. If both sections are present, the rectifying section preferably surmounts the stripping section.

Column C5" is advantageously chosen from distillation columns comprising the two aforementioned sections and the columns that only include one of the two sections. Preferably, column C5" is a distillation column.

Step S5" is therefore preferably a distillation step.

Fraction B, enriched with ethylene, advantageously exits from the top of the column whereas fraction F5", mainly composed of ethane, advantageously exits from the bottom of the column.

The abovementioned step S5" is advantageously carried out at a pressure of at least 5, preferably at least 6 and particularly preferably at least 7 bar absolute. Step S5" is advantageously carried out at a pressure of at most 30, preferably at most 25 and particularly preferably at most 22 bar absolute.

The temperature at which step S5" is carried out is advantageously at least -50, preferably at least -45 and particularly preferably at least -40°C at the bottom of column C5". It is advantageously at most 10, preferably at most 0 and particularly preferably at most -5°C at the bottom of column C5".

The temperature at which step S5" is carried out is advantageously at least -70, preferably at least -65 and particularly preferably at least -60°C at the top of column C5". It is advantageously at most -15, preferably at most -20 and particularly preferably at most -25°C at the top of column C5".

In the process according to the invention, each time the use of a distillation column is mentioned, it may be chosen from plate distillation columns, packed distillation columns, distillation columns with structured packing and distillation columns combining two or more of the abovementioned internals.

The separation steps according to the different embodiment of the process according to the invention are advantageously thermally integrated. The thermal integration is preferably performed either directly, or via one or more refrigeration cycles with temperature levels which are more or less cold, preferably two refrigeration cycles with one at low temperature and the other at medium temperature, or via the combination thereof, more preferably via the combination thereof.

The refrigeration cycles are advantageously based on the compounds containing two carbon atoms, the compounds containing three carbon atoms or their mixtures. Among the compounds containing two carbon atoms, ethylene, ethane and mixtures thereof may be cited. Ethylene is preferred. Among the compounds containing three carbon atoms, propylene, propane and the mixtures thereof may be cited. Propylene is preferred.

The low temperature cycle and the medium temperature cycle are preferably interconnected, that means that the hot source of the low temperature cycle is a cold source of the medium temperature cycle while the hot source of the medium temperature cycle is water from an atmospheric cooling tower. The low temperature cycle preferably uses compounds with 2 carbon atoms and more preferably contains at least 95 mol % of ethylene. The medium temperature cycle preferably uses compounds with 3 carbon atoms and more preferably contains at least 95 mol % of propane or at least 95 mol % of propylene. More preferably, the medium temperature cycle contains at least 95 mol % of propylene.

In the process according to the invention, the fourth and fifth embodiments are preferred.

The chlorination reaction is advantageously carried out in a liquid phase (preferably mainly DCE) containing a dissolved catalyst such as FeCl₃ or another Lewis acid. It is possible to advantageously combine this catalyst with cocatalysts such as alkali metal chlorides. A pair which has given good results is the complex of FeCl₃ with LiCl (lithium tetrachloroferrate - as described in Patent Application NL 6901398).

The amounts of FeCl₃ advantageously used are around 1 to 30 g of FeCl₃ per kg of liquid stock. The molar ratio of FeCl₃ to LiCl is advantageously of the order of 0.5 to 2.

In addition, the chlorination process is preferably performed in a chlorinated organic liquid medium. More preferably, this chlorinated organic liquid medium, also called liquid stock, mainly consists of DCE.

The chlorination process according to the invention is advantageously performed at temperatures between 30 and 150°C. Good results were obtained regardless of the pressure both at a temperature below the boiling point (chlorination under subcooled conditions) and at the boiling point itself (chlorination at boiling point).

When the chlorination process according to the invention is a chlorination process under subcooled conditions, it gave good results by operating at a temperature which was advantageously greater than or equal to 50°C and preferably greater than or equal to 60°C, but advantageously less than or equal to 80°C and preferably less than or equal to 70°C, and with a pressure in the gaseous phase advantageously greater than or equal to 1 and preferably greater than or equal to 1.1 bar absolute, but advantageously less than or equal to 20, preferably less than or equal to 10 and particularly preferably less than or equal to 6 bar absolute.

A process for chlorination at boiling point may be preferred to usefully recover the heat of reaction. In this case, the reaction advantageously takes place at a temperature greater than or equal to 60°C, preferably greater than or equal to 70°C and particularly preferably greater than or equal to 85°C, but advantageously less than or equal to 150°C and preferably less than or equal to 135°C, and with a pressure in the gaseous phase advantageously greater than or equal to 0.2, preferably greater than or equal to 0.5, particularly preferably greater than or equal to 1.1 and more particularly preferably greater than or equal to 1.3 bar absolute, but advantageously less than or equal to 10 and preferably less than or equal to 6 bar absolute.

The chlorination process may also be a hybrid loop-cooled process for chlorination at boiling point. The expression "hybrid loop-cooled process for chlorination at boiling point" is understood to mean a process in which cooling of the reaction medium is carried out, for example, by means of an exchanger immersed in the reaction medium or by a loop circulating in an exchanger, while producing in the gaseous phase at least the amount of DCE formed. Advantageously, the reaction temperature and pressure are adjusted for the DCE produced to leave in the gaseous phase and for the remainder of the heat from the reaction medium to be removed by means of the exchange surface area.

Fraction A containing the ethylene and also the chlorine (itself pure or diluted) may be introduced, together or separately, into the reaction medium by any known device. A separate introduction of fraction A may be advantageous in order to increase its partial pressure and facilitate its dissolution which often constitutes a limiting step of the process.

The chlorine is added in a sufficient amount to convert most of the ethylene and without requiring the addition of an excess of unconverted chlorine. The chlorine/ethylene ratio used is preferably between 1.2 and 0.8 and particularly preferably between 1.05 and 0.95 mol/mol.

The chlorinated products obtained contain mainly DCE and also small amounts of by-products such as 1,1,2-trichloroethane or small amounts of ethane or methane chlorination products. The separation of the DCE obtained from the stream of products derived from the chlorination reactor is carried out according to known modes and in general makes it possible to exploit the heat of the chlorination reaction.

The unconverted products (methane, carbon monoxide, nitrogen, oxygen and hydrogen) are then advantageously subjected to an easier separation than what would have been necessary to separate pure ethylene starting from the initial mixture.

The oxychlorination reaction is advantageously performed in the presence of a catalyst comprising active elements including copper deposited on an inert support. The inert support is advantageously chosen from alumina, silica gels, mixed oxides, clays and other supports of natural origin. Alumina constitutes a preferred inert support.

Catalysts comprising active elements which are advantageously at least two in number, one of which is copper, are preferred. Among the active elements other than copper, mention may be made of alkali metals, alkaline-earth metals, rare-earth metals and metals from the group consisting of ruthenium, rhodium, palladium, osmium, iridium, platinum and gold. The catalysts containing the following active elements are particularly advantageous : copper/magnesium/potassium, copper/magnesium/sodium; copper/magnesium/lithium, copper/magnesium/caesium, copper/magnesium/sodium/lithium, copper/magnesium/potassium/lithium and copper/magnesium/caesium/lithium, copper/magnesium/sodium/potassium, copper/magnesium/sodium/caesium and copper/magnesium/potassium/caesium. The catalysts described in Patent Applications EP-A 255 156, EP-A 494 474, EP-A-657 212 and EP-A 657 213, incorporated by reference, are most particularly preferred.

The copper content, calculated in metal form, is advantageously between 30 and 90 g/kg, preferably between 40 and 80 g/kg and particularly preferably between 50 and 70 g/kg of catalyst.

The magnesium content, calculated in metal form, is advantageously between 10 and 30 g/kg, preferably between 12 and 25 g/kg and particularly preferably between 15 and 20 g/kg of catalyst.

The alkali metal content, calculated in metal form, is advantageously between 0.1 and 30 g/kg, preferably between 0.5 and 20 g/kg and particularly preferably between 1 and 15 g/kg of catalyst.

The Cu:Mg:alkali metal(s) atomic ratios are advantageously 1:0.1-2:0.05-2, preferably 1:0.2-1.5:0.1-1.5 and particularly preferably 1:0.5-1:0.15-1.

Catalysts having a specific surface area, measured according to the BET method with nitrogen that is advantageously between 25 m²/g and 300 m²/g, preferably between 50 and 200 m²/g and particularly preferably between 75 and 175 m²/g, are particularly advantageous.

The catalyst may be used in a fixed bed or in a fluidized bed. This second option is preferred. The oxychlorination process is operated under the range of the conditions usually recommended for this reaction. The temperature is advantageously between 150 and 300°C, preferably between 200 and 275°C and most preferably from 215 to 255°C. The pressure is advantageously above atmospheric pressure. Values of between 2 and 10 bar absolute gave good results. The range between 4 and 7 bar absolute is preferred. This pressure may be usefully adjusted in order to attain an optimum residence time in the reactor and to maintain a constant rate of passage for various operating speeds. The usual residence times range from 1 to 60 s and preferably from 10 to 40 s.

The source of oxygen for this oxychlorination may be air, pure oxygen or a mixture thereof, preferably pure oxygen. The latter solution, which allows easy recycling of the unconverted reactants, is preferred.

The reactants may be introduced into the bed by any known device. It is generally advantageous to introduce the oxygen separately from the other reactants for safety reasons. These safety reasons also require the gaseous mixture leaving the reactor or recycled thereto to be kept outside the limits of inflammability at the pressures and temperatures in question. It is preferable to maintain a so-called rich mixture, that is to say containing too little oxygen relative to the fuel to ignite. In this regard, the abundant presence (> 2 vol %, preferably > 5 vol %) of hydrogen would constitute a disadvantage given the wide range of inflammability of this compound.

The hydrogen chloride/oxygen ratio used is advantageously between 3 and 6 mol/mol. The ethylene/hydrogen chloride ratio is advantageously between 0.4 and 0.6 mol/mol.

The chlorinated products obtained contain mainly DCE and also small amounts of by-products such as 1,1,2-trichloroethane. The separation of the DCE obtained from the stream of products derived from the oxychlorination reactor is carried out according to known modes. The heat of the oxychlorination reaction is generally recovered in vapour form which can be used for the separations or for any other purpose.

The unconverted products such as methane and ethane are then subjected to an easier separation than that which would have been necessary to separate pure ethylene starting from the initial mixture.

The DCE obtained by chlorination or by oxychlorination of ethylene may then be converted to VC.

The invention therefore also relates to a process for the manufacture of VC. To this effect, the invention relates to a process for the manufacture of VC according to which :
a) a low value residual gas is subjected to a series of treatment steps in low value residual gas recovery unit in order to remove the contaminants present therein and to obtain a mixture of products containing ethylene and other constituents;
b) the said mixture of products is separated into a fraction enriched with compounds which are lighter than ethylene, containing part of the ethylene (fraction A), into a fraction enriched with ethylene (fraction B) and into a heavy fraction (fraction C);
c) fraction A is conveyed to a chlorination reactor and fraction B to an oxychlorination reactor, in which reactors most of the ethylene present in the fractions A and B is converted to DCE;
d) the DCE obtained is separated from the streams of products derived from the chlorination and oxychlorination reactors; and
e) the DCE obtained is subjected to a pyrolysis, thus producing the VC.

The particular conditions and preferences defined for the process for the manufacture of DCE according to the invention apply to the process for the manufacture of VC according to the invention.

The conditions under which the pyrolysis may be carried out are known to persons skilled in the art. This pyrolysis is advantageously obtained by a reaction in the gaseous phase in a tubular oven. The usual pyrolysis temperatures are between 400 and 600°C with a preference for the range between 480°C and 540°C. The residence time is advantageously between 1 and 60 seconds with a preference for the range from 5 to 25 seconds. The rate of conversion of the DCE is advantageously limited to 45 to 75 % in order to limit the formation of by-products and the fouling of the tubes of the oven. The following steps make it possible, using any known device, to collect the purified VC and the hydrogen chloride to be upgraded preferably to the oxychlorination. Following purification, the unconverted DCE is advantageously conveyed to the pyrolysis oven.

In addition, the invention also relates to a process for the manufacture of PVC. To this effect, the invention relates to a process for the manufacture of PVC according to which :
a) a low value residual gas is subjected to a series of treatment steps in a low value residual gas recovery unit in order to remove the contaminants present therein and to obtain a mixture of products containing ethylene and other constituents;
b) the said mixture of products is separated into a fraction enriched with compounds which are lighter than ethylene, containing part of the ethylene (fraction A), into a fraction enriched with ethylene (fraction B) and into a heavy fraction (fraction C);
c) fraction A is conveyed to a chlorination reactor and fraction B to an oxychlorination reactor, in which reactors most of the ethylene present in the fractions A and B is converted to DCE;
d) the DCE obtained is separated from the streams of products derived from the chlorination and oxychlorination reactors;
e) the DCE obtained is subjected to a pyrolysis, thus producing VC ; and
f) the VC is polymerized to produce PVC.

The particular conditions and preferences defined for the process for the manufacture of DCE and the process for the manufacture of VC according to the invention apply to the process for the manufacture of PVC according to the invention.

The process for the manufacture of PVC may be a mass, solution or aqueous dispersion polymerization process, preferably it is an aqueous dispersion polymerization process.

The expression aqueous dispersion polymerization is understood to mean free radical polymerization in aqueous suspension as well as free radical polymerization in aqueous emulsion and polymerization in aqueous microsuspension.

The expression free radical polymerization in aqueous suspension is understood to mean any free radical polymerization process performed in aqueous medium in the presence of dispersing agents and oil-soluble free radical initiators.

The expression free radical polymerization in aqueous emulsion is understood to mean any free radical polymerization process performed in aqueous medium in the presence of emulsifying agents and water-soluble free radical initiators.

The expression aqueous microsuspension polymerization, also called polymerization in homogenized aqueous dispersion, is understood to mean any free radical polymerization process in which oil-soluble initiators are used and an emulsion of droplets of monomers is prepared by virtue of a powerful mechanical stirring and the presence of emulsifying agents.

An advantage of the process according to the invention is that it recovers and converts a gas stream containing significant amounts of ethylene which was until the invention characterized by a low valorization (low value residual gas).

Another advantage of the process according to the invention is that it does neither comprise cracking steps followed by organic and aqueous quenching steps nor catalytic oxydehydrogenation steps which need important investment which causes an increase in the production costs and which involve the use of expensive hydrocarbon sources.

An advantage of the process according to the invention is that it makes it possible to have, on the same industrial site, a completely integrated process.

## Claims

1. Process for the manufacture of at least one ethylene derivative compound starting from a low value residual gas according to which :
a) the low value residual gas is subjected to a series of treatment steps in a low value residual gas recovery unit in order to remove the contaminants present therein and to obtain a mixture of products containing ethylene and other constituents;
b) the said mixture of products is separated into a fraction enriched with compounds which are lighter than ethylene, containing part of the ethylene (fraction A), into a fraction enriched with ethylene (fraction B) and into a heavy fraction (fraction C);
c) fraction A and fraction B are conveyed to the manufacture of at least one ethylene derivative compounds.

2. Process according to Claim 1 for the manufacture of one ethylene derivative compound which is DCE.

3. Process according to Claim 2, according to which :
a) the low value residual gas is subjected to a series of treatment steps in a low value residual gas recovery unit in order to remove the contaminants present therein and to obtain a mixture of products containing ethylene and other constituents;
b) the said mixture of products is separated into a fraction enriched with compounds which are lighter than ethylene, containing part of the ethylene (fraction A), into a fraction enriched with ethylene (fraction B) and into a heavy fraction (fraction C);
c) fraction A is conveyed to a chlorination reactor and fraction B to an oxychlorination reactor, in which reactors most of the ethylene present in the fractions A and B is converted to 1,2-dichloroethane; and
d) the 1,2-dichloroethane obtained is separated from the streams of products derived from the chlorination and oxychlorination reactors.

4. Process according to any one of Claims 1 to 3, **characterized in that** the low value residual gas is a refinery off-gas.

5. Process according to Claim 4, **characterized in that** the refinery off-gas is generated in at least one fluid catalytic cracking unit.

6. Process according to any one of Claims 1 to 5, **characterized in that** the low value residual gas comprises from 3 to 60 % by weight of ethylene.

7. Process according to any one of Claims 1 to 6, according to which the low value residual gas is **characterized by** a combustible gas content such that the lower heating value of the gas is comprised between 20 and 75 MJ/kg.

8. Process according to any one of Claims 1 to 7, **characterized in that** fraction B contains from 40 % to 99.5 % by volume of ethylene relative to the total volume of fraction B.

9. Process according to any one of Claims 1 to 8, **characterized in that** fraction A contains a content by volume of ethylene such that it represents from 10 % to 90 % of the content by volume of ethylene of fraction B.

10. Process according to any one of Claims 1 to 9, **characterized in that** fraction C is burnt or valorised chemically.

11. Process for the manufacture of vinyl chloride according to which :
a) a low value residual gas is subjected to a series of treatment steps in low value residual gas recovery unit in order to remove the contaminants present therein and to obtain a mixture of products containing ethylene and other constituents;
b) the said mixture of products is separated into a fraction enriched with compounds which are lighter than ethylene, containing part of the ethylene (fraction A), into a fraction enriched with ethylene (fraction B) and into a heavy fraction (fraction C);
c) fraction A is conveyed to a chlorination reactor and fraction B to an oxychlorination reactor, in which reactors most of the ethylene present in the fractions A and B is converted to DCE;
d) the DCE obtained is separated from the streams of products derived from the chlorination and oxychlorination reactors; and
e) the DCE obtained is subjected to a pyrolysis, thus producing the VC.

12. Process for the manufacture of polyvinyl chloride according to which :
a) a low value residual gas is subjected to a series of treatment steps in a low value residual gas recovery unit in order to remove the contaminants present therein and to obtain a mixture of products containing ethylene and other constituents;
b) the said mixture of products is separated into a fraction enriched with compounds which are lighter than ethylene, containing part of the ethylene (fraction A), into a fraction enriched with ethylene (fraction B) and into a heavy fraction (fraction C);
c) fraction A is conveyed to a chlorination reactor and fraction B to an oxychlorination reactor, in which reactors most of the ethylene present in the fractions A and B is converted to DCE;
d) the DCE obtained is separated from the streams of products derived from the chlorination and oxychlorination reactors;
e) the DCE obtained is subjected to a pyrolysis, thus producing VC ; and
f) the VC is polymerized to produce PVC.
